Europäisches Patentamt

European Patent Office

Office européen des brevets

(11) Publication number: **0 361 625**
**A2**

## (12) EUROPEAN PATENT APPLICATION

(21) Application number: **89202479.5**

(22) Date of filing: **29.09.89**

(51) Int. Cl.5: **G01N 33/12**

(30) Priority: **30.09.88 NL 8802404**

(43) Date of publication of application:
**04.04.90 Bulletin 90/14**

(84) Designated Contracting States:
**AT BE CH DE ES FR GB GR IT LI LU NL SE**

(71) Applicant: **MEYN B.V.**
**Noordeinde 68**
**NL-1511 AE Oostzaan(NL)**

(72) Inventor: **Meyn, Cornelis**
**Haal 62**
**NL-1511 AS Oostzaan(NL)**

(74) Representative: **de Vries, Johannes Hendrik**
**Fokke et al**
**Octrooibureau Los en Stigter B.V. P.O. Box**
**20052**
**NL-1000 HB Amsterdam(NL)**

(54) **Method and apparatus for examining food products by means of irradiation.**

(57) A method is presented for examining food products (11) for undesired ingredients by means of irradiation. This method applies a laser beam (10) which scans the food products according to a predetermined pattern. Variations in the local intensity of the laser beam shining through the food products then indicate the presence of undesired ingredients. This method can be carried out by an apparatus which preferably comprises two parabolic mirrors (4, 5), a laser (9) emitting a laser beam (10) such as to originate from the focus of one of said mirrors and a detection mean (8) positioned in the focus of the other mirror. The food products (11) are moved between the mirrors, for example by a conveyor belt (1, 2).

fig.1

## Method and apparatus for examining food products by means of irradiation

The invention relates to a method for examining food products such as meat undesired ingredients, such as bone fragments, by means of irradiation.

In consequence to the increased demand for a larger assortment of food products, such as meat, fish and poultry, an always increasing automation occured in the respective sectors of industry. With such an automation, of which an example is given by the mechanical filleting of chickens' fillet, a higher production capacity is obtained than would be the case without automation. However, a disadvantage of such an automation is that a final check of the food produkt is required, for example on the presence of bone fragments or the like.

A known method for examining food products by means of irradiation uses X-rays. However, on a large scale applying X-rays for examining food products is commercially unattractive, for the consumer does not like to buy food products which have been treated with X-rays. A possible obligitary indication on a final product that has been examined using X-rays would have very negative consequences on the commercial success of such a food product.

It is an object of the invention to provide a method for examining food products by means of irradiation not having this disadvantage and not being thought of in a negative way by the consumer.

As a result the method according to the invention is characterized in that for the irradiation a laser beam is applied which scans the food product to be examined according to a predetermined pattern and wherein the local intensity of the laser beam shining through the food product is determined for detecting the undesired ingredients.

The application of a laser beam for the irradiation of food products has absolutely no hazards for the public health. Further it appeared that a laser beam penetrates good through food products such as meat, fish and the like. The determined local intensity of the laser beam shining through the food product is substantially proportional with the density of the meat or the undesired ingredients contained therein, such that determined variations in intensity represent a direct indication of undesired ingredients in the food product. By feeding the determined local intensity value to a computer whichs takes into account the specific used wave length of the laser beam, the substance of the food product to be irradiated as well as the substance of the expected ingredients (bone, cartilage, fishbones or the like), it can be determined whether undesired ingredients are present in the food product.

According to a preferred embodiment of the method according to the invention the laser beam scans the food product line by line.

By means of such a line by line scan the computer can create an image of the local intensity variations, so that a picture can be created of the present undesired ingredients.

According to another preferred embodiment of the method according to the invention the laser beam moves to and fro along the food product, wherein the food product is moved over a short distance perpendicularly to the direction of motion of the laser beam whenever said laser beam is in or near to one of its outermost positions.

By the combination of the to and fro movement of the laser beam and the stepwise advancement of the food product this food product is scanned entirely line by line.

The invention further relates to an apparatus for carrying out the method according to the invention. This apparatus is characterized in that it comprises a laser means for moving the laser beam to and fro along the food product to be examined as well as a detection means for determining the intensity of the laser beam shining through the food product.

According to a preferred embodiments of the apparatus according to the invention it comprises a conveyor for the food product to be examined as well as two singularly curved parabolic mirrors, respectively positioned above and below the conveyor, of which the parabolic axes extend mutually in parallel and substantially perpendicularly to the plane of motion of the conveyor, wherein the laser is positioned such that the laser beam is movable to and fro along the surface of one of the parabolic mirrors, however always originates from the focus of this parabolic mirror, whereas the detection means is positioned in the focus of the other parabolic mirror and wherein the conveyor near to the plane described by the laser beam is transmissable for said laser beam.

The described position of the parabolic mirrors and the position of the laser and the detection means make it possible that one single laser beam can cover a plane intersecting the food product to be examined. Like this it is possible to carry out a line by line scan of the food product.

It is advantageous if, according to a further embodiment of the apparatus according to the invention, the laser is stationary and directed such that the laser beam hits a mirror positioned in the focus of said one parabolic mirror and which is pivotable to and fro. Like this the laser beam is in a simple way moved to and fro over the surface of the one parabolic mirror whereas however the laser itself can be stationary.

If finally the conveyor comprises at least two conveyor belts adjoining each other with a gap at the plane of motion of the laser beam, the food product can be moved through the apparatus without the conveyor presenting an obstruction for the laser beam.

Hereafter the invention will be elucidated by means of the drawing, in which an embodiment of the apparatus according to the invention is illustrated.

Figure 1 shows schematically a perspective view of an embodiment of the apparatus according to the invention, and

Figure 2 shows the operational principle of the apparatus according to the invention.

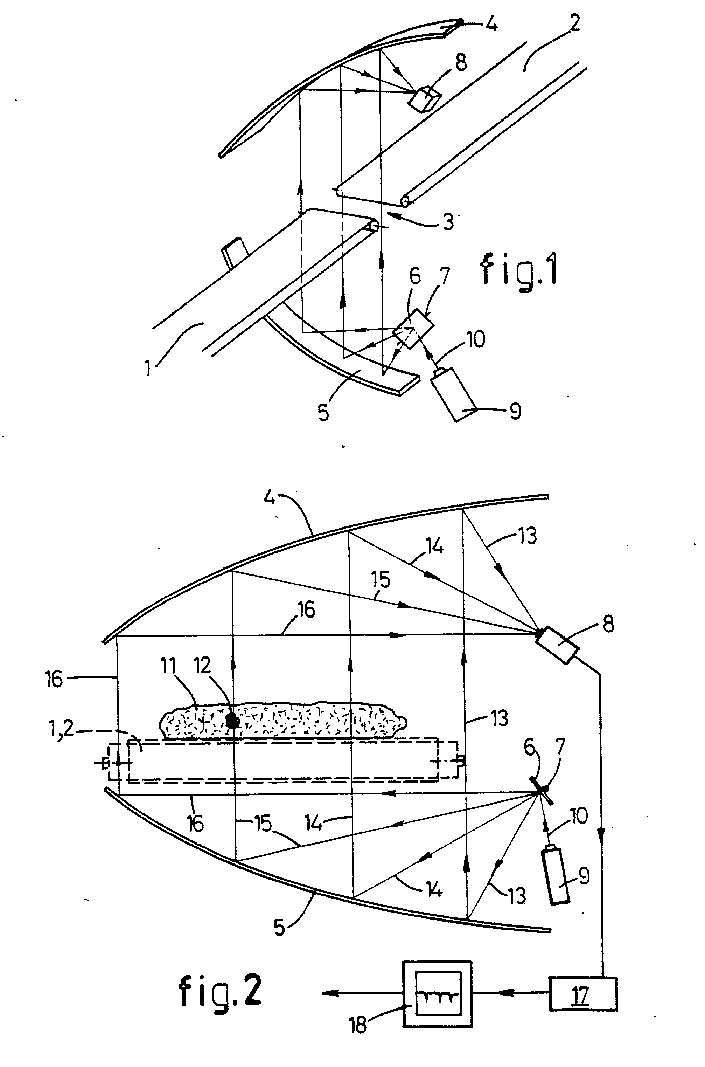

Firstly the apparatus shown in figure 1 comprises a conveyor for the food product to be examined with two conveyor belts 1, 2. These two conveyor belts 1, 2 are aligned and have between them a gap 3.

Above and below the conveyor respective singularly curved parabolic mirrors 4 and 5 are positioned. The position of both parabolic mirrors 4, 5 is such that their parabolic axes extend mutually in parallel and substantially perpendicularly to the plane of motion of the conveyor 1, 2.

In the focus of the lower parabolic mirror 5 a mirror 6 is positioned which, in a way not shown further, is pivotable to and fro about a pivot axis 7. In the focus of the upper parabolic mirror a detection means 8 is positioned.

Moreover, figure 1 shows that the apparatus further comprises a laser 9 which is directed such that the emitted laser beam 10 hits the pivotable mirror 6 substantially in its pivot axis 7. Contrary to the mirror 6 the laser 9 is positioned stationary.

In figure 2 again several parts of the apparatus according to the invention are visible: conveyor 1, 2, upper and lower parabolic mirrors 4 and 5, respectively, mirror 6 pivotable about pivot axis 7, detection means 8 and laser 9. It is clearly visible now that the laser beam 10, which originates from the laser 9, hits the mirror 6 substantially in its pivot axis 7. Moreover in figure 2 a food product 11 to be examined is represented now, in which an undesired ingredient, for example a bone fragment 12, is contained.

As noted before the laser beam 10 hits the pivotable mirror 6. Dependent on the present position of the mirror the bounced back laser beam will hit the lower parabolic mirror 5 on different locations. In figure 2 this is indicated for a number of different locations by means of laser beams 13-16. Because the mirror 6 is positioned in the focus of the lower parabolic mirror 5 the laser beams 13-16 will after hitting this parabolic mirror 5 be directed in parallel with each other, in the present case vertically.

Simultaneously the laser beams 13-16 penetrate dependent from their position the food product 11 to be examined. In the represented position laser beam 15 hits the bone fragment 12 in the food product 11.

Next the laser beams 13-16 hit the upper parabolic mirror 4 and are focused in the detection means 8 positioned in the focus of this parabolic mirror 4. In the present case this detection means 8 is a light sensitive element.

The intensity variations of the incoming laser beams (dependent on the density of the respective food product portion through which the laser beam penetrated) determined by the detection means 8 are processed by a processing unit 17 connected to the detection means 8, such that an indication 18 is obtained of the presence of undesired ingredients. During this processing by means of the processing unit 17 the conveying velocity of the conveyor 1, 2 and the motion of the mirror 6 are taken into account. Like this one can determine which measured intensity belongs to what section of the food product to be examined.

Although above the invention is elucidated by means of an apparatus using two parabolic mirrors it is possible too to chose a configuration which operates without parabolic mirrors. Essential is that for the irradiation a laser beam is applied which scans the food product to be examined according to a predetermined pattern and wherein continuously the local intensity of the laser beam shining through the food product is determined for detecting the undesired ingredients.

The invention is not limited to the embodiment described before which can be varied widely within the scope of the invention.

## Claims

1. Method for examining food products (11) such as meat for undesired ingredients, such as bone fragments (12), by means of irradiation, **characterized** in that for the irradiation a laser beam (10) is applied which scans the food product (11) to be examined according to a predeterminated pattern and wherein the local intensity of the laser beam shining through the food product is determined for detecting the undesired ingredients.

2. Method according to claim 1, **characterized** in that the laser beam (10) scans the food product (11) line by line.

3. Method according to claim 2, **characterized** in that the laser beam (10) moves to and fro along the food product (11), wherein the food product is moved over a short distance perpendicularly to the direction of motion of the laser beam whenever said laser beam is in or near to one of its outermost positions.

4. Apparatus for carrying out the method according to one of the claims 1-3, **characterized** in that it comprises a laser (9) means (4, 5, 6) for moving the laser beam (10) to and fro along the food product (11) to be examined as well as a detection means (8) for determining the intensity of the laser beam shining through the food product.

5. Apparatus according to claim 4, **characterized** in that it comprises a conveyor (1,2) for the food product (11) to be examined as well as two singularly curved parabolic mirrors (4, 5), respectively positioned above and below the conveyor, of which the parabolic axes extend mutually in parallel and substantially perpendicularly to the plane of motion of the conveyor, wherein the laser (9) is positioned such that the laser beam (10) is movable to and fro along the surface of one (5) of the parabolic mirrors, however always originates from the focus of this parabolic mirror (5), whereas the detection means (8) is positioned in the focus of the other parabolic mirror (4) and wherein the conveyor (1, 2) near to the plane described by the laser beam (10) is transmissable for said laser beam.

6. Apparatus according to claim 5, **characterized** in that the laser (9) is stationary and directed such that the laser beam (10) hits a mirror (6) positioned in the focus of said one parabolic mirror (5) and which is pivotable to and fro.

7. Apparatus according to claim 4 or 5, **characterized** in that the conveyor comprises at least two conveyor belts (1, 2) adjoining each other with a gap (3) at the plane of motion of the laser beam (10).

fig.1

fig.2